# EUROPEAN PATENT APPLICATION

(11) **EP 3 763 380 A1**
(43) Date of publication of application: **13.01.2021**
(21) Application number: 18908604.4
(22) Date of filing: 19.11.2018
(51) Int. Cl.: A61K 38/28, A61K 31/4015, A61K 31/4375, A61P 25/28

(54) **METHOD FOR TREATING ENCEPHALOPATHY**

(30) Priority: 06.03.2018 KZ 20180146
(71) Applicant: Lurye, Arman Zhenisovich, Almaly district, Almaty, 050000 (KZ)
(72) Inventor: Lurye, Arman Zhenisovich, Almaly district, Almaty, 050000 (KZ)
(74) Representative: Petniunaite, Jurga
(86) International application number: PCT/KZ2018/000017
(87) International publication number: WO 2019/172737

(57) **Abstract**

The invention relates to medicine, specifically neurology, and can be used to treat patients who have encephalopathy of vascular, traumatic, inflammatory or other origin. In a method for treating encephalopathy by means of drug therapy, according to the invention, a patient's blood glucose level is lowered to 3.4-3.6 mmol/L by intravenous administration of insulin, then solutions of preparations in a single dose are administered intravenously and consecutively with a 30-second interval: first a solution of a vascular preparation having a pharmacological effect, then a solution of a preparation having a direct nootropic effect, then a solution of a peptide preparation and then a solution of a metabolic preparation, wherein one treatment is performed per week, with the total number of treatments being four procedures. In the method for treating encephalopathy, a solution of Cavinton or vinpocetine or trental or pentoxifylline is administered as the vascular preparation having a pharmacological effect, a solution of piracetam or Nootropil or Mexidol or Ceraxon is administered as the preparation having a direct nootropic effect, a solution of Cortexin or Cerebrolysin is administered as the peptide preparation, and a solution of Actovegin or Solcoseryl is administered as the metabolic preparation. The proposed method for treating encephalopathy provides a stable clinical effect by increasing the nootropic, vasoactive and neurotropic effectiveness of the medicinal preparations administered.

## Description

### FIELD OF INVENTION

The invention relates to medicine, particularly, to neurology, and can be used for the treatment of patients with encephalopathy of the vascular, traumatic, inflammatory, and other origin.

### BACKGROUND ART

There is a known method for treatment of chronic diseases of the brain, namely toxic encephalopathy, including the use of piracetam in combination with physiotherapy in the form of transcranial electrical stimulation of the brain, while piracetam is administered intravenously 10-15 minutes before the start of transcranial electrical stimulation of the brain, which is carried out in two stages, duration of 15-20 minutes each, the first stage at an average frequency of 77 Hz, the second stage at an average frequency of 10 Hz (patent RU2362596, classes: A61N1/00, A61P25/00, published on 27.07.2009). For patients using said treatment method, the headaches, dizziness are reduced, and sleeping is improved.

There is also a known method of treating discirculatory encephalopathy by means of complex drug therapy, in which the administration of vinpocetine, riboxin, vitamin B6, as well as ceruloplasmin is used to the patient, and ceruloplasmin is administered daily for 8-10 days intravenously at a rate of 30-35 drops per minute at a dose 100-200 mg in the form of a solution containing 100 mg of ceruloplasmin 200 ml isotonic with sodium chloride solutions (patent RU2242989, class: A61 K38/00, A61P9/10, published on: 08.20.2004). According to the authors, the method provides an increase in the number of patients with an improvement in their condition and the achievement of greater degree of headache reduction.

However, the known methods of treating encephalopathy are not effective enough, since, for example, the restoration of the speech of patients, the restoration of the sensitivity and mobility of the limbs, the ease of the processes of memorizing and reproducing information, improving mood and attention, have not been recorded.

### SUMMARY

The objective of the present invention is to develop a more effective method for the treatment of encephalopathy, with the provision of a stable clinical effect by increasing the nootropic, vasoactive and neurotrophic efficacy of the administered medicamental drugs.

For this purpose, in the method of treating encephalopathy by drug therapy, according to the invention, the patient's blood glucose is lowered to 3.4-3.6 mmol/L by the administration of insulin intravenously, then the drug solutions are administered intravenously sequentially at intervals of 30 seconds, first a vascular solution a drug with a pharmacological effect, then a solution of a drug of direct nootropic action, then a solution of a peptide drug and then a solution of a metabolic drug, with one therapy per week, the total number of therapies is four procedures.

In the method of treating encephalopathy, a solution of Cavinton, or vinpocetine, or Trental, or pentoxifylline is administered as a vascular drug with pharmacological action; a solution of piracetam or Nootropil or Mexidol or Ceraxon is administered as a direct nootropic drug; a peptide drug is administered a solution of Cortexin or Cerebrolysin; and a solution of Actovegin or Solcoseryl is administered as a metabolic preparation.

### DETAILED DESCRIPTION OF THE INVENTION

Nootropics are drugs that have a specific positive effect on higher integrative functions of the brain. They improve mental activity, stimulate cognitive functions, learning and memory, increase the brain's resistance to various damaging factors, including extreme stress and hypoxia. In addition, nootropics have the ability to reduce neurological deficits and improve corticosubcortical connections. The nootropic effect of the drug can be either primary (direct effect on the nerve cell) or secondary, due to improved cerebral blood flow and microcirculation, antiaggregant and antihypoxic effects. That is, drugs can have not only a direct, but also an indirect nootropic effect.

During therapy, drugs of the following groups were used:
1. Vascular with pharmacological action: vasodilating, antiaggregatory, improving microcirculation, normalizing the rheological properties of blood, improving cerebral blood flow and metabolism, including in the brain tissue: trental, quinton, pentoxifylline, vinpocetine;
2. Drugs of direct nootropic action - improving brain functions that affect the metabolic conditions of brain cells: piracetam, Ceraxon, Nootropil, Mexidol;
3. Peptide preparations - a group of polypeptide compounds with nootropic, neuroprotective, antioxidant and tissue-specific action: Cortexin, Cerebrolysin;
4. Metabolic drugs that stimulate regeneration processes, increase glucose utilization and recovery in ischemic areas: Actovegin, Solcoseryl.

Using small doses of insulin to create short-term insignificant hypoglycaemia up to a glucose level in the patient's blood of 3.4-3.6 mmol/l by intravenous administration of insulin and, by injecting intravenously and sequentially with an interval of 30 seconds, solutions of these groups, managed to achieve significant positive dynamics in patients with various types of encephalopathy. During the decrease in blood sugar and simultaneous administration of drugs, the metabolic activity of the brain changes, which is manifested by significant positive changes in patients after therapy.

After four procedures, a significant positive effect is noted, manifested in the relief of symptoms of encephalopathy, namely: restoration of speech, restoration of sensitivity and mobility of the extremities, elimination of dizziness, headaches, ease of the processes of memorization and reproduction of information, reduction of nervousness and anxiety. The indicated significant improvement in the state of health, which provides an increase in the quality of life, has allowed many patients (in accordance with age) to return to work. The treatment effect is long-lasting, which is confirmed by the observation of patients for the past 5 years.

There is a known method of insulinocomatous therapy for intensive therapy of psychoses, for example, acute schizophrenic psychosis (A. I. Nelson "A Short Guide for Doctors on the Use of the Method of Insulinocomatosis Therapy", 2004). Patients with the help of insulin are entered into a coma under the conditions of psychoreanimatology.

It is known to use the symptoms of hypoglycaemia in a method of treating malignant tumours, including chemotherapy, according to which short-acting insulin is administered in a dose of 8-10 IU and, when symptoms of hypoglycaemia are achieved, chemotherapy is administered simultaneously with a 40% glucose solution in a volume of 200-250 ml (patent RU2272647).

In the treatment of encephalopathy, the authors are not aware of insulin therapy.

The proposed method for the treatment of encephalopathy is as follows. The procedure is performed on an empty stomach, the patient is connected to the system, first a small amount of insulin is injected intravenously to a blood sugar of 3.4-3.6 mmol/l, then the drug solutions are administered intravenously at a 30-second interval into the system through a port of a peripheral catheter: single dose of vascular preparations: vinpocetine or Cavinton, or Trental, or pentoxifylline to improve cerebral blood flow and rheological properties of blood, then a single dose of direct nootropic drugs first action: piracetam or Nootropil, Ceraxon or Mexidol, then a single dose of peptide drugs: Cortexin or Cerebrolysin, then a single dose of metabolic drugs - stimulation tori tissue regeneration: Actovegin or Solcoseryl. Conducted 1 such therapy per week. The total number of therapies is four.

The procedure is tolerated by patients without any inconvenience. Positive changes in patients are observed on the second day after the procedure.

Over the past five years, observations have been made of 25 patients with encephalopathies of various origins. Patients prevailed with problems of vascular genesis as well as post-traumatic and multifactorial prevail.

Assessment of treatment results was carried out according to the following factors:
- the dynamics of patient complaints;
- neurovisual examination;

Examinations were carried out after the 4th session of therapy and were repeated 6 months after the last session.

The positive results obtained were persistent for 6 months after the therapy, afterwards, after half a year there was a return of some symptoms. Therefore, therapy was repeatedly carried out in the amount of 4 sessions, and again a stable result was obtained for a period of next 5-7 months.

As examples, information from patient history records is provided. Unfortunately, the choice of objective markers for the diagnosis of encephalopathy is rather scarce. The main criterion remains neuro-visual examination and subjective patient data.

In therapy, various drugs of the same groups were used, taking into account the individual characteristics of the patients.

**Example 1.** Patient Saule T., 57-year-old. Complaints of numbness of the left leg and left arm, speech impairment, persistent headaches like migraines, tinnitus.

Diagnosis: Left-sided ischemic stroke, left-side hemiparesis, speech dysfunction. Encephalopathy.

She had been receiving standard therapy by several courses. Physiotherapy and exercise therapy. Without changes.

Started therapy by the proposed method: NaCl 0.9% 200 ml was connected intravenously, insulin was injected to a blood glucose level of 3.4-3.6 mmol/l, then the preparations were started intravenously sequentially: 1 ml - 5 mg vinpocetine solution, then with an interval of 30 seconds - a solution of piracetam 5 ml of 20%, then with an interval of 30 seconds, a solution of Cerebrolysin 215.2 mg/ml - 2 ml, then with an interval of 30 seconds a solution of Actovegin 200 mg - 5 ml was introduced. At this point, the therapy session was completed.

Conducted 1 such therapy per week. The total number of therapies is 4. At the end of the course, neuro-visual examination reveals restoration of the range of limb movements, stability in the Romberg position, symmetry of tendon reflexes and muscle strength of the limbs.

Subjectively, the patient indicated a significant improvement. Speech was completely restored, the sensitivity and mobility of the limbs were restored. Headaches completely gone. Patient indicates increased energy levels, ease of processes of memorization and reproduction of information. Reduced nervousness and anxiety.

For preventive purposes, after 6 months, 4 sessions of therapy were repeated.

Over 4 years of observation - without negative dynamics.

**Example 2.** Patient Zoya K., 66-year-old. Complaints of headache, general weakness, dizziness, impaired consciousness (turbidity, fainting), impaired spatial orientation, sleep disturbance, memory impairment.

Diagnosis: Condition after multiple microstrokes, atrophy of the cerebral cortex, king-king syndrome, encephalopathy of 3^{rd} stage.

Has been receiving standard therapy for a year. State with negative dynamics. Aimed at obtaining a disability.

Started therapy bv the proposed method: NaCl 0.9% 200 ml was connected intravenously, insulin was injected to a blood glucose level of 3.4-3.6 mmol/l, 15 minutes later, then the following drugs were started intravenously: a 1 ml - 5 mg solution of Cavinton was introduced then, with an interval of 30 seconds, a solution of Nootropil 5 ml of 20%, with an interval of 30 seconds, a solution of Cortexin 10 mg, then with an interval of 30 seconds a solution of Actovegin 200 mg - 5 ml. At this point, the therapy session was completed.

Conducted 1 such therapy per week. The total number of therapies is 4.

At the end of the course, the restoration of the functions of the vestibular apparatus is observed - stability in the Romberg position, ease in orientation in space appeared. Normalization of cognitive functions of the brain. Working ability was fully restored.

Subjectively indicates a significant improvement. Restored sleep, memory, appetite.

Coordination has fully recovered, depression has passed. Headaches completely disappeared. Energy has appeared. It is noted the ease of the processes of memorization and reproduction of information.

For preventive purposes, after 6 months, 4 sessions of therapy were repeated. For 4 years - without negative dynamics.

**Example 3.** Patient Vera 3., 80-year-old. Complaints of general weakness, shortness of breath, severe dizziness, tinnitus, imbalance, sleep disturbance, memory impairment, depressive mood.

Diagnosis: Vertebro-basilar insufficiency, discirculatory encephalopathy of stage 2.

Has been receiving standard therapy for the past 15 years. State with negative dynamics.

Started therapy by the proposed method: NaCl 0.9% 200 ml was connected intravenously, insulin was injected to a blood glucose level of 3.4-3.6 mmol/l, then preparations were started intravenously: a solution of vinpocetine 1 ml - 5 mg, then administered with an interval of 30 seconds a solution of Nootropil 5 ml 20%, then with an interval of 30 seconds - a solution of Cortexin 10 mg, then with an interval of 30 seconds a solution of Actovegin 200 mg - 5 ml was introduced. At this point, the therapy session was completed. Conducted 1 such therapy per week. The total number of therapies is 4.

At the end of the course, restoration of the functions of the vestibular apparatus, stability in the Romberg position, and ease of movement appeared. Improved cognitive functions of the brain. Shortness of breath almost disappeared. Increased energy levels. Subjectively notes a significant improvement. Dizziness stopped. Shortness of breath is significantly reduced, depression has passed. Tinnitus ceased to be noticed.

For preventive purposes, after 6 months, 4 sessions of therapy were repeated. During the last 2 years after therapy - without negative dynamics.

**Example 4.** Patient Svetlana L., 50-year-old. Complaints of general weakness, severe dizziness, imbalance, sleep disturbance, memory impairment, depressive mood.

Diagnosis: Brain cavernoma, tetraparesis, motor aphasia, pelvic organ dysfunction, vestibular syndrome, encephalopathy.

Has been receiving standard therapy for the past 2 years. Condition with improvement. The vestibular syndrome and cognitive impairment persist.

Started therapy by the proposed method: NaCl 0.9% 200 ml was connected intravenously, insulin was injected to a blood glucose level of 3.5 mmol/l, then preparations were administered intravenously sequentially: 1 ml of 5 mg Cavinton solution was introduced, then a solution of pyrrhine was injected with an interval of 30 seconds. Acetam 5 ml of 20%, then with an interval of 30 seconds - a solution of Cortexin 10 mg, then with an interval of 30 seconds a solution of Actovegin 200 mg - 5 ml was introduced. At this point, the therapy session was completed. Conducted 1 such therapy per week. The total number of therapies is 4.

At the end of the course, functions of the vestibular apparatus and speech were restored; depressive syndrome, fatigue - stopped. Pelvic organ function regained control. Improving cognitive functions of the brain. Increasing energy levels.

Subjectively notes a significant improvement in mood, general condition. Began to control the bladder. The dizziness has stopped. Depression has passed. There was a feeling of lightness in the head.

For prophylactic purposes, after 9 months, 4 sessions of therapy were repeated. Over the past 2 years after therapy - without negative dynamics.

**Example 5.** Patient Karen V., 67-year-old. Complaints of weakness, fatigue, pain in the heart, gait unsteadiness, frequent headaches, impaired concentration, high blood pressure, tinnitus.

Diagnosis: Hypertension of 2 stage, Dyscirculatory encephalopathy.

Started therapy by the proposed method: NaCl 0.9% 200 ml was connected intravenously, insulin was injected to a blood glucose level of 3.4-3.6 mmol/l, then the preparations were started intravenously sequentially: Trental solution 20 mg/ml - 5 ml, then at intervals 30 seconds - a solution of Ceraxon 500 mg - 4 ml, then with an interval of 30 seconds, a solution of Cerebrolysin 215.2 mg / ml - 2 ml, then with an interval of 30 seconds a solution of Actovegin 200 mg - 5 ml was introduced. At this point, the therapy session was completed.

Conducted 1 such therapy per week. The total number of therapies is 4.

At the end of the course, neuro-visual examination shows stability in the Romberg position, symmetry of tendon reflexes and muscle strength of the limbs, normalization of blood pressure to a level of 120/80 mm Hg.

Subjectively, the patient notes a significant improvement. He stopped worrying about pains in the heart area. Increased vigour, activity. The appearance of a feeling of lightness and joy. Headaches stopped. He indicates an increase in the level of energy, ease of the processes of memorization and reproduction of information. Reduction of nervousness and anxiety.

Over 3 years of observation - without negative dynamics.

**Example 6.** Patient Ramon, 60-year-old. Complaints of a tiredness, frequent colds, weakness, sleep disturbance, mood swings, memory impairment and concentration. Apathy.

Diagnosis: Encephalopathy of mixed origin.

Started therapy by the proposed method: NaCl 0.9% 200 ml was connected intravenously, insulin was injected to a blood glucose level of 3.4-3.6 mmol/l after 15 minutes, then the administration of drugs was started intravenously: pentoxifylline solution 20 mg/ml - 5 mg, then with an interval of 30 seconds - a solution of Mexidol 5% - 2 ml, with an interval of 30 seconds, a solution of Cerebrolysin 215.2 mg/ml - 2 ml, then with an interval of 30 seconds a solution of Solcoseryl 42.5 mg/ml - 2 ml was introduced. At this point, the therapy session was completed.

Conducted 1 such therapy per week. The total number of therapies is 4.

At the end of the course, a significant improvement in overall tone was noted, sleep was restored, and a stable-positive mood was noted. Normalization of cognitive functions of the brain. Apathy fully passed.

Subjectively indicates a significant improvement in his condition. Indicates the ease of falling asleep and waking up, the processes of memorizing information have noticeably improved.

For 2 years - without negative dynamics.

**Example 7.** Patient Marina Z., 58-year-old. Complaints of headaches, impaired memory, attention, flickering flies in front of the eyes, periodically impaired coordination when walking and poor writing.

Diagnosis: Discirculatory encephalopathy of 2 stage, destruction of the vitreous body.

Repeatedly with this diagnosis has been receiving standard therapy. State with dynamics with a gradual deterioration.

Started therapy by the proposed method: NaCl 0.9% 200 ml was connected intravenously, insulin was injected to a blood glucose level of 3.4-3.6 mmol/l, then preparations were started intravenously: Cavinton solution 1 ml - 5 mg, then administered with an interval of 30 seconds a solution of Ceraxon 500 mg - 4 ml, then with an interval of 30 seconds - a solution of Cortexin 10 mg, then with an interval of 30 seconds a solution of Solcoseryl 42.5 mg / ml - 2 ml was introduced. At this point, the therapy session was completed.

Conducted 1 such therapy per week. The total number of therapies is 4.

At the end of the course, stability in the Romberg position is noted, lightness and clarity in writing appeared. Improving cognitive functions of the brain. Increasing energy levels. Subjectively, a noticeable improvement is noted, the flies before the eyes completely disappeared.

Over the past 2 years after therapy without negative dynamics. Two years later, one fly appeared before eyes (there were five).

**Example 8.** Patient Marat R., 80-year-old. No complaints. Apathetic. He comes into contact with difficulty. Objectively - right-sided central hemiparesis, urinary incontinence, emotional stinginess, lack of exercise, hypomimia. From the anamnesis - in 2008 suffered an ischemic stroke, six months later - another. For 7 years he was treated in Israel, Korea, Europe and Kazakhstan. Last 3 years without noticeable improvements.

Diagnosis: Condition after suffering ischemic strokes in 2008, right-sided central hemiparesis, 3 stage discirculatory encephalopathy.

Had been receiving standard therapy for the past 7 years. The last 3 years, the condition is without improvement.

Started therapy by the proposed method: NaCl 0.9% 200 ml was connected intravenously, insulin was injected to a blood glucose level of 3.5 mmol/l, then preparations were administered intravenously in series: Trental solution 20 mg/ml - 5 ml was administered, then a Nootropil solution was administered with an interval of 30 seconds 5 ml of 20%, then with an interval of 30 seconds - a solution of Cortexin 10 mg, then with an interval of 30 seconds a solution of Solcoseryl 42.5 mg/ml - 2 ml was introduced. At this point, the therapy session was completed.

Conducted 1 such therapy per week. The total number of therapies is 4.

Starting from 3 weeks, he began to control urination, an emotional response appeared to surrounding events, he began to actively communicate with the clinic staff, his gait became dynamic, he began to smile and joke.

At the end of the course, restoration of the functions of the vestibular apparatus, restoration of speech and cognitive functions of the brain are noted. Completely restored control over the function of the pelvic organs.

Subjectively notes a significant improvement in memory, mood, general condition. Began to control the bladder. The patient has become social.

Over the past 3 years after therapy - without negative dynamics.

**Example 9.** Patient B-ov B., 68-year-old. Complaints of apathy, including depression, loss of interest in life, work, family, general total fatigue, emotional emptiness. Sleep disturbance, memory impairment.

Diagnosis: Hypertension 1 stage, discirculatory encephalopathy of 2 stage.

Started therapy by the proposed method: NaCl of 0.9% 200 ml was connected intravenously, insulin was injected to a blood glucose level of 3.4-3.6 mmol/l, then preparations were started intravenously in series: Trental solution 20 mg/ml - 5 ml, then with an interval of 30 seconds Mexidol solution was introduced 5% - 2 ml, then with an interval of 30 seconds - a solution of Cortexin 10 mg, then with an interval of 30 seconds, a solution of Solcoseryl 42.5 mg/ml - 2 ml was introduced. At this point, the therapy session was completed.

Conducted 1 such therapy per week. The total number of therapies is 4.

Upon completion of the course, restoration of cognitive functions, moods, and activity is noted. Completely gone apathy and depression. There was energy and creativity, and sleep was restored. He returned to his senior management position.

Over the past 2 years after therapy - without negative dynamics.

## Claims

1. A method of treating encephalopathy by drug therapy, **characterized in that** the glucose in the blood of the patient is lowered to 3.4-3.6 mmol/l by the administration of insulin intravenously, then the drug solutions are administered intravenously at intervals of 30 seconds in a single dose, first, a solution of a vascular drug with a pharmacological effect, then a solution of a drug of direct nootropic action, then a solution of a peptide drug and then a solution of a metabolic drug, with one therapy per week, total number of therapies is four treatments.

2. A method of treating encephalopathy by drug therapy according to claim 1, **characterized in that** a solution of Cavinton or vinpocetine or trental or pentoxifylline is administered as a vascular drug with a pharmacological action, a solution of piracetam or Nootropil or Mexidol is administered as a direct nootropic drug or Ceraxon, a solution of Cortexin or Cerebrolysin is administered as a peptide preparation, a solution of Actovegin or Solcoseryl is administered as a metabolic preparation.
